(19) Europäisches Patentamt
European Patent Office
Office européen des brevets

(11) Publication number: **0 109 078**
**B1**

(12) # EUROPEAN PATENT SPECIFICATION

(45) Date of publication of patent specification: **04.05.88**

(21) Application number: **83111327.9**

(22) Date of filing: **12.11.83**

(51) Int. Cl.⁴: **G 01 N 33/76,**
G 01 N 33/531,
G 01 N 33/535

(54) **Immunochemical assay of human chorionic gonadotropin and reagent therefor.**

(30) Priority: **15.11.82 JP 200897/82**

(43) Date of publication of application:
**23.05.84 Bulletin 84/21**

(45) Publication of the grant of the patent:
**04.05.88 Bulletin 88/18**

(84) Designated Contracting States:
**BE CH DE FR GB IT LI NL SE**

(56) References cited:
**EP-A-0 037 110**
**EP-A-0 049 898**

**ENDOCRINOLOGY, vol. 104, January-June 1979, SHUJI MATSUURA et al. "A Human Chorionic Gonadotropin-Specific Antiserum against Synthetic Peptide Analogs to the Carboxyl-Terminal Peptide of its Beta-Subunit" pages 396-401**

**EUROPEAN JOURNAL OF BIOCHEMISTRY, vol. 101, no. 2, November 1979 SHINJI YOSITAKE et al. "Conjugation of Glucose Oxidase from Aspergillus niger and Rabbit Antibodies Using N-Hydroxysuccinimide Ester of N-(4-Carboxycyclohexylmethyl)-Maleimide" pages 395-399**

(73) Proprietor: **Takeda Chemical Industries, Ltd.**
**27, Doshomachi 2-chome Higashi-ku**
**Osaka-shi Osaka, 541 (JP)**

(72) Inventor: **Kondo, Koichi**
**6-213, 2 Taishibashi 3-chome**
**Asahi-ku Osaka 535 (JP)**

(74) Representative: **von Kreisler, Alek, Dipl.-Chem. et al**
**Deichmannhaus am Hauptbahnhof**
**D-5000 Köln 1 (DE)**

(56) References cited:
**HORMONE AND METABOLIC RESEARCH, vol. 8, no. 3, May 1976 J.C. GARAUD et al. "Unusual Specifities of Antibodies to Glucagon-Glutaraldehyde-Albumin Conjugates" pages 241-243**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 109 078 B1

**Description**

This invention relates to an immunochemical assay of human chorionic gonadotropin (hereinafter sometimes referred to as "hCG").

Heretofore, two methods have been proposed for enzyme immunoassay (hereinafter sometimes referred to briefly as "EIA") of hCG, as follows:

(1) Competitive method: The assay is accomplished by allowing a reagent containing a definite amount of hCG labelled with an enzyme and a sample containing an unknown amount of hCG to become bound to a known amount of an anti-hCG antibody competitively, assaying the enzymic activity of that portion of enzyme which has been bound to the antibody or remains unbound and comparing the assay result with the result obtained in the same manner with a known amount of hCG.

(2) Sandwich method: The assay is accomplished by trapping a sample containing an unknown amount of hCG with an anti-hCG antibody supported on a carrier, allowing an enzyme-labelled antibody to be bound to the resulting complex and assaying the enzymic activity.

The present inventor has already found that the use of a certain β-D-galactosidase as labeling agent and a certain antibody in the above-mentioned competitive method (1) enables microassay of hCG with high sensitivity and high specificity (cf. European Patent Application Publication EP—A—37,110). The present inventor has also found that, in the above-mentioned sandwich method (2), an EIA which comprises using an antibody supported on the carrier and an antibody to which the labeling agent is coupled, wherein two antibodies are different antibodies which are not overlapping in antigen-determining site and one of these different antibodies is directed against the ß-subunit of human chorionic gonadotropin, is highly sensitive, highly accurate and highly specific (cf. European Patent Publication EP—A—49,898).

European Journal of Biochemistry, Vol. 101, No. 2, November 1979, pages 395 to 399 discloses the coupling of glucose oxidase and rabbit antibody by employing the N-hydroxysuccinimide ester of N-(4-carboxycyclohexylmethyl)-maleimide as a coupling agent.

However, even by these methods, it is difficult to assay hCG at levels of 0.1 to 2 mIU or below. Accordingly, a still more highly sensitive assay method has been required for more decisive diagnosis of malignant tumor and observation of the course of such tumor as well as study of the physiological meaning of hCG in normal human subjects.

Under these circumstances, the present inventor conducted further investigations and found that, in EIA by the sandwich method which uses the above-mentioned specific antibody, the use of an antibody-enzyme conjugate obtained by coupling an anti-hCG antibody to peroxidase enables a highly sensitive, highly accurate microassay. Continued studies based on the above finding have now led to completion of the present invention.

The present invention relates to an immuno-chemical assay of human chorionic gonadotropin at a level of 0.1 to 2m IU involving the use of an antibody supported on a carrier, an antigen and an antibody labeled with a labeling agent, characterized in that said supported antibody and the labeled antibody are dissimilar antibodies which are not overlapping with each other in antigen-determinant position, that the supported antibody is directed against the β-subunit of human chorionic gonadotropin, that said labeling agent is horseradish peroxidase, and that the peroxidase is coupled with the corresponding antibody employing a compound represented by the formula:

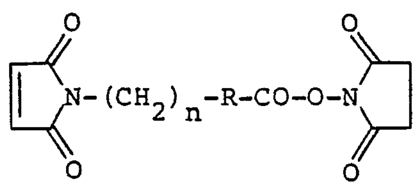

[I]

wherein n is an integer of 0 to 5 and R is a chemical bond or a divalent 6-membered cyclic saturated or unsaturated hydrocarbon residue. Moreover, this invention relates to immunochemical assay reagents for use in an assay of human chorionic gonadotropin at a level of 0.1 to 2 m IU which comprises component (a) a reagent consisting of a conjugate which is prepared by coupling horseradish peroxidase with an antibody employing the compound of the formula [I] and component (b) an antibody supported on a carrier, the antibody being not overlapping in antigen-determinant position with the antibody to be coupled with peroxidase and being directed against the β-subunit of human chorionic gonadotropin.

The carrier used in the present invention includes, among others, beads of gels {for example, agarose gel [e.g. Sepharose®4B, Sepharose®6B (Pharmacia Fine Chemicals, Sweden)], dextran gel [e.g. Sephadex®G75, Sephadex®G100, Sephadex®G200 (Pharmacia Fine Chemicals)], polyacrylamide gel [e.g. Biogel®P30, Biogel®P60, Biogel®P100 (Bio-Rad Laboratories, U.S.A.)]}, particles of cellulose [for example, Avicel® (Asahi Chemical Industry Co., Ltd. Japan), ion exchange cellulose (e.g. diethylaminoethyl-cellulose, carboxymethyl-cellulose)], physical adsorbents [for example, glass (e.g. glass beads, glass rods, aminoalkyl-glass beads, aminoalkyl-glass rods), silicone rubbers, styrene resin (e.g. polystyrene beads,

polystyrene granules)], ion exchange resins {for example, weakly acid cation exchange resins [e.g. Amberlite®IRC-50 (Rohm and Haas, U.S.A.), Zeocarb®226 (Permutit, West Germany)], weakly basic anion exchange resins [e.g. Amberlite®IR-4B (Rohm and Haas) and Dowex®3 (Dow Chemical, U.S.A.)]}.

In accordance with this invention, the antibody supported on a carrier and the antibody coupled with a labeling agent are two dissimilar antibodies which are not overlapping with each other in antigen-determinant position, and the antibody to be supported on the carrier is an antibody specifically reactive with the β-subunit of hCG.

As examples of the antibody specifically reactive with the β-subunit of hCG, there is mentioned (1) an antibody described in Endocrinology, vol. 104 (1979), P.396. Thus, an hCG-specific peptide at the C-terminal of hCG-β subunit and a carrier protein such as bovine albumin or bovine-thyroglobulin are condensed in the presence of a water-soluble carbodiimide reagent such as 1-ethyl-3-(3-dimethyl-aminopropyl) carbodiimide, and using the resulting condensate and Freund's complete adjuvant or incomplete adjuvant, a warm-blooded animal such as rabbit is immunized a plurality of times to produce an antibody. This procedure gives an antiserum which reacts specifically to hCG.

(2) An hCG-specific anti-hCG antibody described in European Patent Application Publication EP—A—37,110 is mentioned. The antibody is prepared as follows: The peptide of the formula [VIII]:

$$H\text{-}R_1\text{-Pro-Ser-Asp-Thr-Pro-Ile-Leu-Pro-Gln-OH} \qquad [VIII]$$

wherein $R_1$ is a partial peptide of 1 to 14 amino acid residues including 14-Gly of the peptide $Ala^1\text{-}Pro^2\text{-}Pro^3\text{-}Pro^4\text{-}Ser^5\text{-}Leu^6\text{-}Pro^7\text{-}Ser^8\text{-}Pro^9\text{-}Ser^{10}\text{-}Arg^{11}\text{-}Leu^{12}\text{-}Pro^{13}\text{-}Gly^{14}$,
as immobilized on a carrier, is contacted with a body fluid containing an anti-hCG antibody, and the anti-hCG antibody specifically absorbed is separated by elution.

(3) Furthermore, as an antibody which reacts specifically with the β-subunit of hCG, there may be mentioned an antibody prepared by condensing a peptide of the formula [VIII] mentioned above with a carrier protein in the presence of glutaraldehyde (hereafter briefly, GLA), inoculating a warm-blooded animal other than man with the resulting condensate to produce an antibody and recovering the same (European Patent Application Publication EP—A—49,898).

The carrier protein referred to above is a substance which is coupled with a hapten (a substance of low molecular weight) for producing an antibody to the hapten such as a peptide which, alone, cannot induce formation of an antibody, and includes such proteins as bovine serum albumin, bovine gamma-globulin, bovine thyroglobulin, tetanus toxoid, hemocyanin and polyamino acid.

The coupling of the peptide of the formula [VIII] with a carrier protein in the presence of GLA can be conducted by the conventional method [e.g. "Hormone and Metabolic Research", vol 8 (1976) P.241]. The relative amount of peptide [VIII] and carrier protein is preferably 1:1 to 2:1, and the reaction pH of about 7.3 gives satisfactory results in many cases. The reaction time is somewhere between 2 and 6 hours and a reaction time of about 3 hours is usually appropriate. The condensation product thus obtained is dialyzed against water of about 4°C, lyophilized and stored as usual.

The condensation product thus obtained is used to inoculate a warm-blooded animal other than man.

The warm-blooded animal other than man, which is used in the production of the above hCG-specific antibody, includes, among others, mammalian warm-blooded animals (e.g. rabbit, sheep, rat, mouse, guinea pig, cattle, horse, pig) and avian species (e.g. chicken, pigeon, duck, goose, quail).

To inoculate the condensation product into such a warm-blooded animal other than man, the condensation product is used in an amount sufficient to produce the desired antibody. For example, 2 mg per dose of the condensation product is emulsified with equal volumes (1 ml) of physiological saline and Freund's complete adjuvant and the emulsion is injected subcutaneously into dorsal sites and rear foot pads of a rabbit for a total of 5 times at intervals of 4 weeks. The above procedure yields the desired antibody in many instances.

The antibody thus produced in the body of the warm-blooded animal can be harvested as follows. Thus, in the case of a rabbit, for instance, blood is withdrawn from the ear vein and centrifuged to separate the serum normally at a time between 7 to 12 days after the last immunization.

The carrier for supporting the immobilized antibody in the assay of hCG may be any of the carriers mentioned above.

The coupling of the carrier with the antibody may be effected by the conventional method. For example, the cyanogen bromide method as well as the GLA method described in "Taisha" (Metabolism and Disease), vol. 8 (1971), P.696 published by Nakayama Shoten Inc., Japan may be mentioned. As a more expedient procedure, the antibody may be physically adsorbed on the surface of the carrier.

As examples of the peptide of the formula [VIII], there is mentioned a C-terminal fragment peptide [IX] of hCG-β (123-145) represented by the formula: H-Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-OH. The fragment is produced, for example, by a method described in European Patent Application Publication EP—A—37,110.

Throughout this specification, when abbreviations are used to amino acids, peptides, etc., they are either the abbreviations according to IUPAC-IUB Commission on Biochemical Nomenclature or those of common use in this field of art. The following is a partial list of the abbreviations. It is to be understood that when optical isomers exist in regard to amino acids, etc., L-forms are meant unless otherwise indicated.

Ala : alanine
Pro : proline
Ser : serine
Leu : leucine
Arg : Arginine
Gly : glycine
Asp : aspartic acid
Thr : threonine
Ile : isoleucine
Gln : glutamine

The various peptides which are employed for the production of the specific antibody according to the present invention can be produced by procedures known *per se.* While both of the solid-phase and the liquid-phase methods of synthesis may be employed, the latter method is more often advantageous. Such methods for peptide synthesis include those described in the literature, e.g. Schröder and Lübke: The Peptides, vol. 1 (1966), Academic Press, New York, U.S.A. and Izumiya et al.: "Peptide Gosei" (Peptide Synthesis) (1975), Maruzen Co., Ltd., Japan, namely the azide method, chloride method, acid anhydride method, mixed acid anhydride method, DCC method, active ester method, Woodward Reagent K method, carbodiimidazole method, reduction-oxidation method and DCC-additive (e.g. HONB, HOBt, HOSu) method.

The coupling of the carrier with the specific antibody may be effected by the conventional method. For example, the cyanogen bromide method as well as the GLA method described in the aforementioned "Metabolism and Disease", vol. 8 (1971), P.696 published by Nakayama Shoten Inc., Japan may be mentioned. As a more expedient procedure, the antibody may be physically adsorbed on the surface of the carrier.

The antigen in the assay according to the invention is the assay target, namely hCG.

Referring to the antibody labeled with a labeling agent to be used in the practice of the invention, the antibody therein is nonspecifically reactive with hCG and has an antigenic determinant which does not overlap with any of the antigen-determinant position of the above-mentioned antibody supported on a carrier.

An example of the antibody nonspecifically reactive to hCG is the antibody which shows cross-reactivity with other structurally analogous protein hormones such as hLH (human luteinizing hormone), which antibody may be prepared by inoculating a warm-blooded animal other than a human being with a purified hCG derived from human urine in the conventional manner to produce an anti-hCG antibody, subjecting the same antibody to salting-out to recover γ-globulin fraction, subjecting the same fraction to affinity chromatography on a column packed with a solid phase coupled with the C-terminal peptide of hCG-β to recover an effluent and purifying the effluent further by affinity chromatography on a column of a solid phase to which hCG has been coupled.

The labeling agent is horseradish peroxidase extracted from horseradish.

In coupling horseradish peroxidase to the antibody, a compound of the formula:

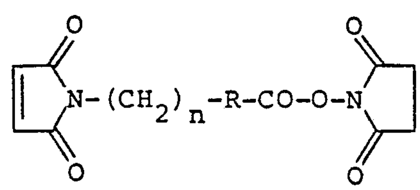

[I]

wherein n and R are defined as above, is used. In the above formula, the divalent 6-membered-cyclic hydrocarbon residue represented by R may be either saturated or unsaturated. Examples of the saturated divalent 6-membered-cyclic hydrocarbon residue are 1,2-, 1,3- and 1,4-cyclohexylene. Examples of the unsaturated 6-membered-cyclic hydrocarbon residue are 1,2-, 1,3- and 1,4-phenylene.

In said compound [I], n is preferably an integer of 1 to 5, and more preferably the integer 1. As the divalent 6-membered-cyclic hydrocarbon residue R, 1,4-cyclohexylene is particularly preferable.

The compound [I] to be used in the method of the present invention can be produced, for example, by the methods described in The Journal of Biochemistry, vol. 79, page 233 (1976), European Journal of Biochemistry, vol. 101, page 395 (1979), Japanese Patent Application Laid-open No. Sho 52 (1977)-85,163, Japanese Patent Application Laid-open No. Sho 52 (1977)-85,164 and German Patent Application Offenlegungsschrift DE—A—2656155 or modifications thereof. Thus, for instance, it can be produced by reacting a maleimide compound of the formula:

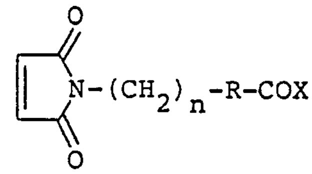

[II]

wherein X is a hydroxyl group or a halogen atom and n and R are as defined above, with a succinimide compound of the formula:

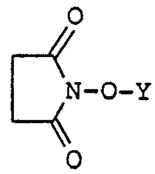

[III]

wherein Y is a hydrogen atom or an alkali metal atom, in the presence of a dehydrating agent or a deacidifying agent. Referring to the above formulas, the halogen atom is, for example, chlorine or bromine, and the alkali metal atom is, for instance, sodium or potassium. Examples of the dehydrating agent to be used in the reaction are sulfuric acid and dicyclohexylcarbodiimide. Pyridine and triethylamine are examples of the deacidifying agent.

The above-mentioned compound [II] can be produced for example, by the method described in Japanese Patent Application Laid-open No. 52 (1977)—85,164 or a modification thereof. Thus, for instance, it can be obtained by subjecting a compound [IV] of the formula:

$$\begin{array}{l} HC—CO—NH—(CH_2)_n—R—COOH \\ \| \\ HC—COOH \end{array} \qquad [IV]$$

wherein n and R are as defined above, to dehydrating ring closure. Said dehydration-ring closure reaction can be effected by gentle heating with dehydrating agent such as acetic anhydride or acetic anhydride plus sodium acetate (anhydrous).

Alternatively, it can also be produced by the method described in Helvetica Chimica Acta, vol. 58 (1975), page 531 or a modification thereof. Thus, for instance, an N-alkoxycarbonylmaleimide [V] of the formula:

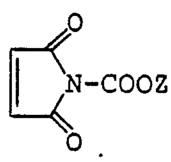

[V]

wherein Z is an alkyl group, with an amino acid [VI] of the formula:

$$NH_2(CH_2)_nRCOOH \qquad [VI]$$

wherein n and R are as defined above, to give a maleimide compound [VII] of the formula:

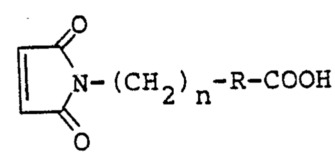

[VII]

wherein n and R are as defined above. Then, a succinimide compound [III] is added and the reaction is carried out in the presence of a dehydrating or deacidifying agent such as mentioned above, to give the compound [I].

Referring to the compound of the formula [V], the alkyl represented by Z is, for example, methyl or ethyl.

The reaction of compound [I] with horseradish peroxidase is accomplished by contacting both with each other in a buffer having a pH of 6 to 8 at a temperature of 10°C to 50°C for 10 minutes to 24 hours. Said buffer is, for example, 0.1 M phosphate buffer (pH 7.0) or 0.05 M phosphate buffer (pH 6.3).

The thus-obtained maleimidated peroxidase can be purified by gel chromatography, for instance. Packing materials adequate for said gel chromatography are Sephadex G—25 (Pharmacia Fine Chemical,

Sweden) and Biogel P—2 (Bio-Rad Laboratories, U.S.A.), among others.

An exemplary method for reacting the maleimidated horseradish peroxidase with the anti-hCG antibody is described below. The anti-hCG antibody IgG or the $F(ab')_2$ fragment obtained by pepsin digestion is reduced in the presence of a mercaptoethylamine and the unreacted material is removed by gel filtration. The resultant anti-hCG antibody IgG or Fab′ is reacted with the maleimidated horseradish peroxidase.

Said reaction can be effected by contacting both with each other in a buffer at a temperature of 0°C to 40°C for 1 to 48 hours. Said buffer is, for example, 0.1 M phosphate buffer containing 5 mM sodium ethylenediamine-tetraacetate (pH 6.0).

The thus-produced horseradish peroxidase-labeled antibody can be purified by gel chromatography, for instance. As the packing material for use in said gel chromatography, there may be mentioned, among others, Ultrogel® AcA (LKB, Sweden) and Sephacryl® S—200 (Pharmacia Fine Chemical, Sweden).

Thus obtained peroxidase-labeled antibody is presumed to have the following structure:

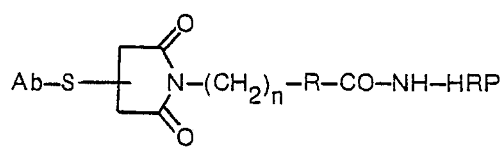

wherein R and n are the same meaning as defined above, Ab means a residue of the antibody (Ab—SH), and HRP is a residue of horseradish peroxidase ($HRP—NH_2$).

The assay procedure according to the present invention is described in more detail hereinbefore.

(1) First, an hCG-containing sample to be assayed is added to the antibody supported on a carrier, whereupon the antigen-antibody reaction takes places. Thereafter, the horseradish peroxidase-coupled anti-hCG antibody IgG or Fab′ obtained in the above manner is added for reaction.

As the hCG-containing sample to be assayed by the enzyme immunoassay method according to the present invention, there may be mentioned urine, serum, plasma, cerebrospinal fluid and various internal organ extracts, among others. Among them, urine, serum and plasma are most frequently used.

(2) A substrate for horseradish peroxidase is added to the reaction product obtained in (1), and the absorbance or fluorescence intensity of the substance produced is measured. The enzymatic activity of the above-mentioned reaction product is known in this manner.

(3) The above serial procedures (1) and (2) is followed with standard solutions containing known amounts of hCG and a standard curve indicating the relationship between the hCG level and the absorbance or fluorescence intensity is constructed in advance.

(4) The hCG content in the test sample is determined by conversion of the absorbance of fluorescence intensity obtained with the test sample containing an unknown amount of hCG using the standard curve.

An assay kit suited for use in immunochemical assay of hCG by the sandwich method according to the present invention mainly comprises:

(1) the antibody supported on a carrier,

(2) the horseradish peroxidase-labeled antibody fragment (Fab′) obtained by the method of the present invention,

(3) a standard hCG,

(4) a buffer for diluting the above reagents (2) and (3) and a test sample (e.g. phosphate or glycine buffer having a pH of 6 to 9 and containing about 10% of sheep serum and about 1% of bovine serum albumin (hereinafter sometimes referred to as "BSA") in each of which serum and proteinic substances are coexisting), and

(5) reagents necessary for determination of the horseradish peroxidase activity (e.g. p-hydroxyphenylacetic acid as a substrate and hydrogen peroxide for fluroescent assay; o-phenylenediamine and hydrogen peroxide for colorimetry; a buffer for dissolving the substrate, preferably phosphate buffer; and an enzymatic reaction terminator solution).

The above kit can be used, for instance, in the following manner:

10 to 200 µl of the standard hCG or test liquid is diluted with the reagent (4). Then, an adequate amount of the reagent (1) is added. After 1 to 48 hours of reaction at 0° to 40°C, the carrier is washed with water and, then, 10 to 300 µl of the reagent (2) is added. After 1 to 48 hours of reaction at 0° to 40°C, the carrier is washed and the activity of horseradish peroxidase on the carrier is determined. Thus, 10 to 1,000 µl of a solution of a peroxidase substrate is added and the enzymic reaction is allowed to proceed at 20°C to 40°C for 0.2 to 24 hours and then terminated, followed by determination of the absorbance or fluorescence intensity of the liquid reaction mixture.

The reagents for immunochemical assay in accordance with the present invention enables a highly sensitive assay of hCG by a simple procedure in a usual clinical laboratory.

When the reagents according to the invention is used, hCG can be assayed with very high sensitivity and accuracy without suffering interference from similar hormones (e.g. hLH: luteinizing hormone). Accordingly, the present invention provides a means very useful also for diagnosis and prognosis of chorionic tumors and other types of hCG-producing tumors, among others.

Brief Description of the Drawings:

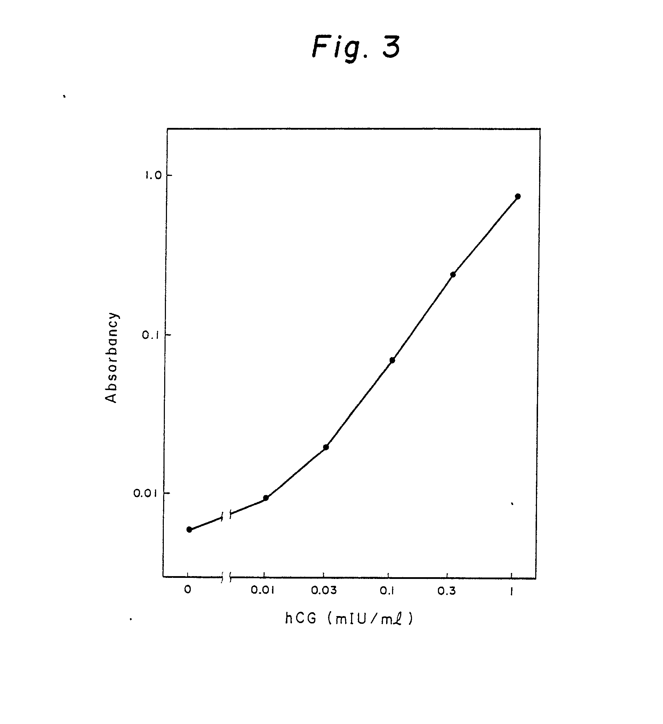

Fig. 1 shows the gel chromatographic elution patterns of the reaction product of horseradish peroxidase with rabbit anti-hCG antibody (Fab' fragment) in Example 1; Fig. 2 shows the standard curves of hCG and hLH in the EIA using the horseradish peroxidase-labeled anti-hCG antibody (Fab' fragment) and the anti-β subunit of hCG-specific antibody-polystyrene bead in Example 1; and Fig. 3 shows the standard curve of hCG in the single-incubation assay in Example 2.

The following Reference Examples and Examples are given to illustrate the present invention in further detail.

## Reference Example 1

Production of anti-hCG antibody (anti-hCG serum):

In 1 ml of physiological saline was dissolved 1 mg of hCG (approx. 10,000 IU/mg) purified from the human urine by the conventional method, and 1 ml of Freund's complete adjuvant [Tachibana et al.: Men-eki-no-Seikagaku (Biochemistry of Immunity), p. 26, Kyoritsu Shuppan Inc. Japan (1967)] was added and stirred well to prepare an emulsion. This emulsion was injected into the bilateral femoral muscles and subcutaneously at several dorsal sites of a rabbit. The above procedure was repeated at intervals of 3 weeks for a total of 5 times and a blood sample was taken one week after the last immunization for a pilot assay. In this manner, there was obtained an anti-serum N305B having an affinity also for the C-terminal fragment peptide [IX] of hCG-β.

## Reference Example 2

Production of the antibody anti-β subunit of hCG:

Five (5) mg of peptide [IX] was dissolved in 8 ml of 0.1 M $NaHCO_3$ containing 0.5 M NaCl. To this solution was added 1 g of BrCN-activated Sepharose 4B previously washed with 1/1,000 N HCl. The mixture was stirred at 5°C overnight. Then, the Sepharose was washed well with the 0.1 M $NaHCO_3$ solution containing 0.5 M NaCl as used above, followed by addition of 10 ml of 0.5 M ethanolamine adjusted to pH 8 with hydrochloric acid. The reaction was conducted at room temperature for one hour, after which time the Sepharose was washed with (1) 0.1 M acetate buffer containing 1 M NaCl (pH 4.0), (2) 0.1 M borate buffer containing 1 M NaCl (pH 8.0) and (3) 0.02 M borate buffer containing 0.15 M NaCl (pH 8.0) in the order mentioned. The Sepharose was then packed into a column.

Eight (8) ml of the anti-hCG serum N305B obtained according to Reference Example 1 was subjected to fractional precipitation with 1.5 g of anhydrous sodium sulfate and the resultant γ-globulin fraction was passed through the above column of peptide [IX]-Sepharose 4B (column size: 0.9 × 4 cm).

The column was washed with 0.02 M borate buffer containing 0.15 M NaCl (pH 8.0) to remove the anti-hCG antibodies showing cross-reactivity with hLH, hFSH (human follicle-stimulating hormone) and hTSH (human thyroid-stimulating hormone). Then, elution was carried out with 0.17 M glycine-HCl buffer (pH 2.3) to recover the specific anti-hCG antibody N305BS having a strong affinity for the C-terminal fragment peptide [IX] of HCG-β (Protein content 1.2 mg).

## Reference Example 3

Production of anti-hCG-β C-terminal fragment peptide [IX] antibody:

In 4 ml of 0.2 M phosphate buffer (pH 7.3) were dissolved 25 mg of the C-terminal peptide [IX] of hCG-β and 50 mg of bovine thyroglobulin (briefly, BTG), followed by addition of 4 ml of 5% aqueous GLA. The mixture was stirred at room temperature for 3 hours, after which it was dialyzed against water at 4°C (2 l of water × 4) and lyophilized to obtain an immunogen. In 0.75 ml of physiological saline was dissolved 1.5 mg of the above hCG-β C-terminal peptide [IX]-BTG conjugate, followed by addition of 0.75 ml of Freund's complete adjuvant. The mixture was stirred well to prepare an emulsion. The emulsion was injected intramuscularly into bilateral femoral muscles and subcutaneously several dorsal sites of a rabbit. The above procedure was repeated four times at intervals of 4 weeks and the blood was collected a week after the last immunization, centrifuged to separate the antiserum. In the above manner, anti-hCG-β C-terminal peptide [IX] serum N313B was obtained.

This antiserum N313B was precipitated with ammonium sulfate in the conventional manner and the resulting γ-globulin fraction was applied to a Sepharose 4B column (0.9 cm dia. × 4 cm long) carrying 2 mg of hCG.

The column was washed with 0.02 M borate buffer containing 0.15 M NaCl (pH 8.0) and elution was carried out with 0.17 M glycine-HCl buffer (pH 2.3), whereby a specific antibody N313BS having a high affinity for hCG was obtained.

## Reference Example 4

Production of nonspecific anti-hCG antibody:

Antiserum N305B obtained in Reference Example 1 was salted out with ammonium sulfate and subjected to affinity chromatography using a column (0.9 cm in diameter and 4 cm in length) of Sepharose 4B coupled with 5 mg of the C-terminal peptide [IX] of hCg-β. An antibody fraction was recovered as the effluent. This fraction was then passed through a column (0.9 cm in diameter and 4 cm in length) of Sepharose 4B coupled with 2 mg of hCG. The column was washed with 0.02 M borate buffer (pH 8.0)

containing 0.15 M NaCl and then eluted with 5 M $MgCl_2$ to give nonspecific antibody N305BG having a high affinity to hCG.

Example 1

(1) Introduction of maleimide group:

In 1 ml of 0.1 M phosphate buffer (pH 7.0), there was dissolved 6 mg of horseradish peroxidase (Boehringer Mannheim, West Germany), followed by addition of 4.8 mg of a coupling agent, MMC (the compound of the formula [I] wherein n=1 and R=1,4-cyclohexylene), dissolved in 50 μl of N,N-dimethylformamide. The mixture was stirred at 30°C for 60 minutes. After the reaction, the resultant precipitate was removed by centrifugation and the supernatant was passed through a Sephadex G-25 column (1.0 × 45 cm), followed by elution with 0.1 M phosphate buffer. A protein-containing fraction was collected and concentrated with a collodion membrane. The thus-prepared maleimidated peroxidase contained 1.0 to 1.2 maleimide groups introduced per one molecular of peroxidase (calculation was made on the assumption that the molecular weight of peroxidase was 40,000 and $E_{1\%}^{280\ nm}$ = 22.75).

(2) Production of maleimidated peroxidase-anti-hCG antibody (Fab' fragment) complex:

To 5 mg of antibody N305BG obtained in Reference Example 4 was added 0.1 mg of pepsin, followed by overnight reaction at 30°C. The reaction mixture was passed through a Sephadex G—150 column (2.5 cm in diameter and 55 cm in length). The thus-purified antibody $F(ab')_2$ fraction was reduced with 2-mercaptoethylamine, followed by purification by gel chromatography using a Sephadex G—25 column to give rabbit anti-hCG antibody (Fab' fragment).

To a solution of 1.5 mg of the maleimidated peroxidase prepared in (1) above in 0.15 ml of 0.1 M phosphate buffer (pH 6.0), there was added 0.15 ml of 0.1 M phosphate buffer (pH 6.0) containing 1.8 mg of the previously obtained anti-hCG antibody (Fab' fragment) and 5 mM sodium ethylenediamine-tetraacetate. After 20 hours of reaction at 4°C, the reaction mixture was subjected to gel chromatography using a column (1.5 × 45 cm) packed with Ultrogel® AcA 44. Elution was performed with 0.1 M phosphate buffer (pH 6.5). The absorbance at 280 nm and enzymatic activity of the eluate were determined. The formation of a horseradish peroxidase-rabbit anti-hCG antibody (Fab' fragment) conjugate was confirmed by the following method.

First, the enzymatic activity was determined by the method of Gilbert et al. [Analytical Chemistry, vol. 40 (1968), page 1256). Thus, each eluate fraction was diluted 1,800 times with 0.1 M phosphate buffer (pH 7.0) containing 0.1% of bovine serum albumin. To 10 μl of each dilution was added 0.25 ml of a 0.5% solution of p-hydroxyphenylacetic acid in 0.05 M sodium acetate buffer (pH 5.0) containing 0.1% of bovine serum albumin, followed by incubation at 30°C for 20 minutes. Thereafter, 0.05 ml of 0.01% hydrogen peroxide was added, followed by reaction at 30°C for 20 minutes. The enzymic reaction was terminated by addition of 2.5 ml of 0.1 M glycine buffer (pH 10.3) and the intensity of fluorescence was measured at 405 nm (excitation light wavelength: 320 nm) regarding the intensity of fluorescence emitted by 1 μg/ml of quinine as 100. The results obtained are shown in Fig. 1. In Fig. 1, —○— indicates the absorbance at 280 nm and —●— indicates the peroxidase activity (in terms of fluorescence intensity). It was confirmed that the formation of the horseradish peroxidase-anti-hCG antibody (Fab' fragment) conjugate was very well recognizable in fraction 38 and neighboring fractions.

(3) Preparation of antibody-coupled solid phase:

To 1,500 polystyrene beads (4.8 mm in diameter, Precision Plastics Ball Co., Chicago, U.S.A.) were added 100 ml of 15 μg/ml solution of specific anti-hCG antibody N305BS or N313BS mentioned in Reference Example 2 or 3, respectively, in 0.01 M NaCl—0.01 M phosphate buffer (pH 8.0). After overnight incubation at 5°C, the beads were washed with 0.05 M phosphate buffer (pH 7.0) containing 0.1% of BSA and stored in the cool until use thereof.

(4) EIA:

The following EIA reagents were used.

(1) The horseradish peroxidase-rabbit anti-hCG antibody (Fab' fragment) complex according to Example 1 (2).

(2) The anti-hCG antibody-coupled polystyrene bead according to Example 1 (3).

(3) Standard hCG and hLH solutions.

(4) Buffer B: a 0.02 M phosphate buffer (pH 7.0) containing 10% normal sheep serum, 1% bovine serum albumin, 0.1% $NaN_3$ and 0.15 M NaCl.

(5) The reagents for determination of peroxidase activity: a 0.1 M citric acid-disodium phosphate buffer (pH 4.8) containing 0.02% hydrogen peroxide and 0.15% o-phenylenediamine and a reaction terminator (1 N-HCl).

Assay

Either hCG or hLH was reacted with the rabbit anti-hCG-coupled polystyrene bead in 200 μl of buffer B at room temperature for 1 day. After the polystyrene bead was washed with water, 300 μl of horseradish peroxidase-rabbit anti-hCG antibody (Fab' fragment) complex was added and the reaction was conducted

at 4°C for 1 day. After washing the polystyrene bead, 500 μl of a 0.1 M citric acid-disodium phosphate buffer (pH 4.8) containing 0.02% hydrogen peroxide and 0.15% o-phenylenediamine was added. The reaction system was allowed to stand at room temperature for 40 minutes, at the end of which time 2 ml of 1 N hydrochloric acid was added to thereby terminate the reaction. The absorbance of the reaction mixture was measured at 492 nm. The standard curves constructed in the above manner are shown in Fig. 2. In Fig. 2, —●— represents the standard curve of hCG for the N305BS-polystyrene bead; —○— the standard curve of hLH for the N305BS-polystyrene bead; —■— the standard curve of hCG for the N313BS-polystyrene bead; and —□— the standard curve of hLH for the N313BS-bound polystyrene bead.

Thus, the assay method of the present invention was highly specific and sensitive to hCG without the interference of cross-reaction with hLH.

Example 2

Expedient assay:

Standard hCG, the peroxidase-anti-hCG antibody (Fab' fragment) complex according to Example 1 (2), and one anti-hCG antibody-N305BS-polystyrene bead according to Example 1 (3) were simultaneously mixed together in 300 μl of a 0.02 M phosphate buffer (pH 7.0) containing 25% normal sheep serum, 1% bovine serum albumin, 0.002% merthiolate and 0.15 M NaCl and reacted at 4°C for 1 day. After the polystyrene bead was washed with water, peroxidase activity was determined by the procedure described in Example 1 (4). As shown in Fig. 3, a satisfactory standard curve of hCG was obtained.

Example 3

m-Maleimidobenzoyl-N-hydroxysuccinimide (MBS) coupling method:

In 0.1 M phosphate buffer (pH 7.0) was dissolved 6 mg of horseradish peroxidase [Boehlinger-Mannheim, West Germany] followed by addition of 4.8 mg of the coupling agent MBS (the compound of the formula [I] wherein n=0, R=m-phenylene) as dissolved in 50 μl of N,N'-dimethylformamide. The reaction was conducted under stirring at 25°C for 30 minutes and the reaction mixture was then passed through a Sephadex G—25 column (1.0 × 45 cm), and eluted with 0.05 M acetate buffer (pH 5.0). The protein-containing fraction was taken and concentrated through a collodion membrane. The number of maleimide groups in the maleimidated peroxidase per one mole of peroxidase was 0.68 to 0.78.

To 5 mg of the antibody N305BG according to Reference Example 4 was added 0.1 mg of pepsin and the reaction was conducted at 30°C overnight. The reaction product was then purified by passing through a Sephadex G—150 column (2.5 cm Dia.× 55 cm long). The resulting antibody $F(ab')_2$ fraction was reduced with 2-mercaptoethylamine and, then, purified by gel chromatography on a Sephadex G—25 column to give a rabbit anti-hCG antibody (Fab' fragment). A solution (0.15 ml) of 1.7 mg of anti-hCG antibody (Fab' fragment) in a 0.1 M phosphate buffer (pH 6.0) containing 5 mM sodium ethylenediaminetetracetate was added to 0.15 ml of a solution containing 1.5 mg of the maleimidated peroxidase prepared above in 0.1 M phosphate buffer (pH 6.0) and the reaction was conducted at 4°C for 20 hours. The reaction mixture was then subjected to gel-chromatography on an Ultrogel AcA 44 column (1.5 × 45 cm) and elution was carried out with 0.1 M phosphate buffer (pH 6.5) to give a peroxidase-rabbit anti-hCG antibody (Fab' fragment) complex.

Assays using this complex by the procedure described in Example 1 (2) and (4) showed that it was highly sensitive, with a very low non-specific adsorption on the solid phase.

The EIA reagents used were as follows.

(1) The anti-hCG antibody N305BS-polystyrene bead according to Example 1 (3).

(2) The peroxidase-rabbit anti-hCG antibody (Fab' fragment) complex according to Example 3.

(3) Standard hCG.

(4) Buffer B [cf. Example 1 (4)].

(5) Reagents for determination of peroxidase activity.

An acetate buffer (pH 5.0) containing 0.5% p-hydroxyphenylacetic acid, a 0.01% hydrogen peroxide solution, and a reaction terminator (0.1 M glycine buffer, pH 10.3).

Example 4

A hCG specific immunochemical assay kit, and the assay of hCG:

Using the hCG immunochemical assay kit and procedure described below, plasma hCG concentration was determined in healthy subjects, ovariectomized patients, women in the ovulation phase, pregnant women, and patients who had undergone carcinectomy for choriocarcinoma.

hCG immunochemical assay kit:

(1) The 4.8 mm (dia.) polystyrene bead coupled with about 1 μg/bead of hCG-specific antibody as obtained according to Example 1 (3).

(2) The horseradish peroxidase-anti-hCG antibody conjugate according to Example 1 (2).

(3) 0 to 100 mIU of standard hCG.

(4) Buffer B [cf. Example 1 (4)] for dilution of the above reagents (1) and (3) and the test sample.

(5) o-Phenylenediamine.

(6) Buffer C for dilution of reagent (2): a 0.1 M phosphate buffer (pH 7.5) containing 0.1% bovine serum

albumin and 0.002% merthiolate.

(7) Buffer D for dilution of reagent (5): a 0.1 M citric acid buffer (pH 4.8) containing 0.02% of hydrogen peroxide and 0.002% of merthiolate.

(8) Reaction terminator: 1 N hydrochloric acid.

(9) Control serum (normal sheep serum).

Procedure:

To 50 µl of the test sample were added 250 µl of reagent (4) and one bead (reagent 1), and the reaction was conducted at room temperature for 1 day. After washing the polystyrene bead with water, 300 µl of reagent (2) as diluted with reagent (6) (about 30 ng as the conjugate) was added and the reaction was conducted at 4°C for 1 day. After the polystyrene bead was washed with water, 500 µl of a 0.15% solution of reagent (5) in reagent (7) was added. The reaction was conducted at room temperature for 40 minutes, at the end of which time 1.5 ml of 1 N hydrochloric acid was added to thereby terminate the reaction. The absorbance was then measured at 492 nm. As to standard hCG solutions, the above procedure was repeated using 200 µl of reagent (4), 50 µl of control serum, and one bead of reagent (1) to construct a standard curve.

The hCG concentrations in the sera of healthy subjects, ovariectomized patients, women in the ovulation phase, pregnant women and patients who had undergone carcinectomy for choriocarcinoma were determined by the procedure set forth hereinbefore. The results are shown in Table 1.

TABLE 1

| Test sample | | Concentration of hCG (mIU/ml) |
|---|---|---|
| Serum of healthy woman | 1 | 0.20 |
| | 2 | 0.25 |
| | 3 | 0.35 |
| | 4 | 0.18 |
| | 5 | 0.27 |
| Serum of ovariectomized woman | 1 | 0.33 |
| | 2 | 0.86 |
| | 3 | 0.54 |
| | 4 | 0.23 |
| | 5 | 0.77 |
| Serum of woman in ovulation phase | 1 | 0.34 |
| | 2 | 0.19 |
| | 3 | 0.25 |
| Serum of pregnant woman | 1 | 1,000 |
| | 2 | 34,000 |
| | 3 | 18,000 |
| Serum of patient who had undergone carcinectomy for choriocarcinoma | 1 | 190 |
| | 2 | 9.8 |
| | 3 | 5.3 |

It will be apparent from Table 1 that very small amounts of hCG in serum, inclusive of the sera of

healthy subjects, can be successfully assayed without the interference of cross-reaction with hLH and other analogous hormones. Moreover, this assay method is very useful as a test of cure in the prognostic management of patients undergoing carcinectomy for choriocarcinoma.

## Claims

1. An immunochemical assay of human chorionic gonadotropin at a level of 0.1 to 2 mIU involving the use of an antibody supported on a carrier, an antigen and an antibody labeled with a labeling agent, characterized in that said supported antibody and the labeled antibody are dissimilar antibodies which are not overlapping with each other in antigen-determinant position, that the supported antibody is directed against the β-subunit of human chorionic gonadotropin, that said labeling agent is horseradish peroxidase, and that the peroxidase is coupled to the antibody being labeled employing a compound represented by the formula:

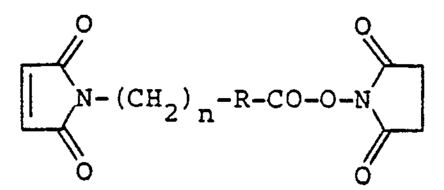

[I]

wherein n is an integer of 0 to 5 and R is a chemical bond or a divalent 6-membered cyclic saturated or unsaturated hydrocarbon residue.

2. An immunochemical assay as claimed in Claim 1, wherein the antibody directed against the β-subunit of human chorionic gonadotropin is an antibody obtained by contacting a body fluid containing an anti-hCG antibody with a peptide of the formula:

H—$R_1$-Pro-Ser-Asp-Thr-Pro-Ile-Leu-Pro-Gln-OH

wherein $R_1$ is a partial peptide of 1 to 14 amino acid residues including 14-Gly of the peptide

$Ala^1$-$Pro^2$-$Pro^3$-$Pro^4$-$Ser^5$-$Leu^6$-$Pro^7$-$Ser^8$-$Pro^9$-$Ser^{10}$-$Arg^{11}$-$Leu^{12}$-$Pro^{13}$-$Gly^{14}$,

immobilized on a carrier, and separating the anti-hCG antibody specifically absorbed by elution.

3. An immunochemical assay as claimed in Claim 2, wherein the peptide is

H-Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-OH.

4. An immunochemical assay as claimed in Claim 1, wherein the antibody directed against the β-subunit of human chorionic gonadotropin is an antibody obtained by conjugating a peptide of the formula:

H—$R_1$-Pro-Ser-Asp-Thr-Pro-Ile-Leu-Pro-Gln-OH

wherein $R_1$ is a partial peptide of 1 to 14 amino acid residues including 14-Gly of the peptide

$Ala^1$-$Pro^2$-$Pro^3$-$Pro^4$-$Ser^5$-$Leu^6$-$Pro^7$-$Ser^8$-$Pro^9$-$Ser^{10}$-$Arg^{11}$-$Leu^{12}$-$Pro^{13}$-$Gly^{14}$,

with a carrier protein in the presence of glutaraldehyde and inoculating the resulting conjugate into a warm-blooded animal other than man to produce an antibody and recovering it.

5. An immunochemical assay as claimed in Claim 4, wherein the peptide is

H-Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-OH.

6. An immunochemical assay as claimed in Claim 1, wherein the compound used in the coupling of horseradish peroxidase to the antibody being labeled is a compound represented by the formula:

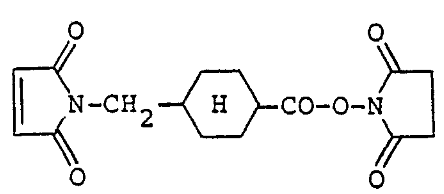

7. An immunochemical assay reagents for use in an assay of human chorionic gonadotropin at a level

of 0.1 to 2 mIU according to Claim 1 which comprises component (a) a reagent consisting of a conjugate which is prepared by coupling horseradish peroxidase to an antibody employing the compound of the formula:

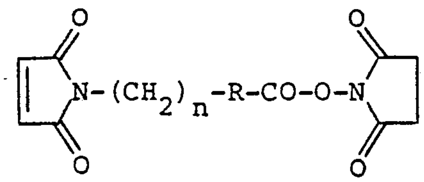

[I]

wherein n is an integer of 0 to 5 and R is a chemical bond or a divalent 6-membered cyclic saturated or unsaturated hydrocarbon residue, and component (b) an antibody supported on a carrier, the antibody being not overlapping in antigen-determinant position with the antibody to be coupled to peroxidase and being directed against the β-subunit of human chorionic gonadotropin.

8. An immunochemical assay reagents as claimed in Claim 7, wherein the antibody directed against the β-subunit of human chorionic gonadotropin is an antibody obtained by contacting a body fluid containing an anti-hCG antibody with a peptide of the formula:

H—$R_1$-Pro-Ser-Asp-Thr-Pro-Ile-Leu-Pro-Gln-OH

wherein $R_1$ is a partial peptide of 1 to 14 amino acid residues including 14-Gly of the peptide

$Ala^1$-$Pro^2$-$Pro^3$-$Pro^4$-$Ser^5$-$Leu^6$-$Pro^7$-$Ser^8$-$Pro^9$-$Ser^{10}$-$Arg^{11}$-$Leu^{12}$-$Pro^{13}$-$Gly^{14}$,

immobilized on a carrier, and separating the anti-hCG antibody specifically absorbed by elution.

9. An immunochemical assay reagents as claimed in Claim 8, wherein the peptide is

H—Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-OH.

10. An immunochemical assay reagents as claimed in Claim 7, wherein the antibody directed against the β-subunit of human chorionic gonadotropin is an antibody obtained by conjugating a peptide of the formula:

H—$R_1$-Pro-Ser-Asp-Thr-Pro-Ile-Leu-Pro-Gln-OH

wherein $R_1$ is a partial peptide of 1 to 14 amino acid residues including 14-Gly of the peptide

$Ala^1$-$Pro^2$-$Pro^3$-$Pro^4$-$Ser^5$-$Leu^6$-$Pro^7$-$Ser^8$-$Pro^9$-$Ser^{10}$-$Arg^{11}$-$Leu^{12}$-$Pro^{13}$-$Gly^{14}$,

with a carrier protein in the presence of glutaraldehyde and inoculating the resulting conjugate into a warm-blooded animal other than man to produce an antibody and recovering it.

11. An immunochemical assay reagents as claimed in Claim 10, wherein the peptide is

H—Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-OH.

12. An immunochemical assay reagents as claimed in Claim 7, wherein the compound used in the coupling of horseradish peroxidase to the antibody being labeled is a compound represented by the formula:

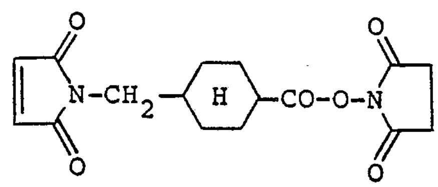

**Patentansprüche**

1. Immunchemische Analyse von Human-Choriongonadotropin bei Werten von 0,1 bis 2 mIE unter Verwendung eines trägerunterstützten Antikörpers, eines Antigens und eines mittels eines Markierungsmittels markierten Antikörpers, dadurch gekennzeichnet, daß der trägerunterstützte Antikörper und der markierte Antikörper ungleichartige Antikörper sind, die einander in bezug auf die antigen-determinante Position nicht überlappen, daß der trägerunterstützte Antikörper gegen die β-Untereinheit des Human-Choriongonadotropins gerichtet ist, daß das Markierungsmittel Meerrettich-Peroxidase mit und daß die Peroxidase mit dem markiert werdenden Antikörper mit Hilfe einer Verbindung der Formel

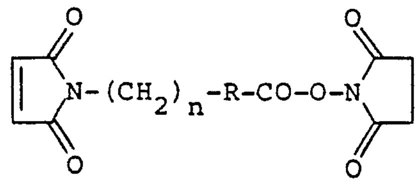

[I]

gekoppelt wird, in der n eine ganze Zahl von 0 bis 5 ist und R eine chemische Bindung oder ein zweiwertiger 6-gliedriger cyclischer gesättigter oder ungesättigter Kohlenwasserstoff-Rest ist.

2. Immunchemische Analyse nach Anspruch 1, bei der der gegen die β-Untereinheit des Human-Choriongonadotropins gerichtete Antikörper ein Antikörper ist, der dadurch erhalten wurde, daß eine einen Anti-hCG-Antikörper enthaltende Körperflüssigkeit mit einem Peptid der Formel

H—$R_1$-Pro-Ser-Asp-Thr-Pro-Ile-Leu-Pro-Gln-OH,

in der $R_1$ ein partielles Peptid mit 1 bis 14 Aminosäure-Resten, einschließlich 14-Gly, des Peptids Ala$^1$-Pro$^2$-Pro$^3$-Pro$^4$-Ser$^5$-Leu$^6$-Pro$^7$-Ser$^8$-Pro$^9$-Ser$^{10}$-Arg$^{11}$-Leu$^{12}$-Pro$^{13}$-Gly$^{14}$, immobilisiert auf einem Träger, in Berührung gebracht wird und der spezifisch absorbierte Anti-hCG-Antikörper durch Elution abgetrennt wird.

3. Immunchemische Analyse nach Anspruch 2, bei der das Peptid

H—Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-OH

ist.

4. Immunchemische Analyse nach Anspruch 1, bei der der gegen die β-Untereinheit des Human-Choriongonadotropins gerichtete Antikörper ein Antikörper ist, der dadurch erhalten wurde, daß ein Peptid der Formel

H—$R_1$-Pro-Ser-Asp-Thr-Pro-Ile-Leu-Pro-Gln-OH,

in der $R_1$ ein partielles Peptid mit 1 bis 14 Aminosäure-Resten, einschließlich 14-Gly, des Peptids Ala$^1$-Pro$^2$-Pro$^3$-Pro$^4$-Ser$^5$-Leu$^6$-Pro$^7$-Ser$^8$-Pro$^9$-Ser$^{10}$-Arg$^{11}$-Leu$^{12}$-Pro$^{13}$-Gly$^{14}$, mit einem Träger-Protein in Gegenwart von Glutaraldehyd konjugiert wird und das resultierende Konjugat einem warmblütigen Tier, jedoch nicht dem Menschen, eingeimpft wird, um einen Antikörper zu erzeugen und diesen zu gewinnen.

5. Immunchemische Analyse nach Anspruch 4, bei der das Peptid

H—Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-OH

ist.

6. Immunchemische Analyse nach Anspruch 1, bei der die beim Koppeln der Meerrettich-Peroxidase mit dem markiert werdenden Antikörper verwendete Verbindung eine Verbindung der Formel

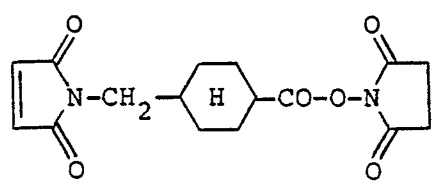

ist.

7. Immunchemische Analysenreagentien zur Verwendung bei einer Analyse von Human-Choriongonadotropin bei Werten von 0,1 bis 2 mIE nach Anspruch 1, umfassend Komponente

(a) ein Reagens bestehend aus einem Konjugat, das hergestellt wird durch Koppeln von Meerrettich-Peroxidase mit einem Antikörper mit Hilfe einer Verbindung der Formel

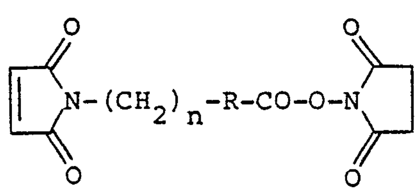

[I]

in der n eine ganze Zahl von 0 bis 5 ist und R eine chemische Bindung oder ein zweiwertiger 6-gliedriger cyclischer gesättigter oder ungesättigter Kohlenwasserstoff-Rest ist, und Komponente

(b) einen trägerunterstützten Antikörper, der in bezug auf die antigen-determinante Position den mit der Peroxidase zu koppelnden Antikörper nicht überlappt und gegen die β-Untereinheit des Human-Choriongonadotropins gerichtet ist.

8. Immunchemische Analysenreagentien nach Anspruch 7, bei denen der gegen die β-Untereinheit des Human-Choriongonadotropins gerichtete Antikörper ein Antikörper ist, der dadurch erhalten wurde, daß eine einen Anti-hCG-Antikörper enthaltende Körperflüssigkeit mit einem Peptid der Formel

$$H—R_1\text{-Pro-Ser-Asp-Thr-Pro-Ile-Leu-Pro-Gln-OH},$$

in der $R_1$ ein partielles Peptid mit 1 bis 14 Aminosäure-Resten, einschließlich 14-Gly, des Peptids $Ala^1$-$Pro^2$-$Pro^3$-$Pro^4$-$Ser^5$-$Leu^6$-$Pro^7$-$Ser^8$-$Pro^9$-$Ser^{10}$-$Arg^{11}$-$Leu^{12}$-$Pro^{13}$-$Gly^{14}$, immobilisiert auf einem Träger, in Berührung gebracht wird und der spezifisch absorbierte Anti-hCG-Antikörper durch Elution abgetrennt wird.

9. Immunchemische Analysenreagentien nach Anspruch 8, bei denen das Peptid

H—Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-OH

ist.

10. Immunchemische Analysenreagentien nach Anspruch 7, bei denen der gegen die β-Untereinheit des Human-Choriongonadotropins gerichtete Antikörper ein Antikörper ist, der dadurch erhalten wurde, daß ein Peptid der Formel

$$H—R_1\text{-Pro-Ser-Asp-Thr-Pro-Ile-Leu-Pro-Gln-OH},$$

in der $R_1$ ein partielles Peptid mit 1 bis 14 Aminosäure-Resten, einschließlich 14-Gly, des Peptids $Ala^1$-$Pro^2$-$Pro^3$-$Pro^4$-$Ser^5$-$Leu^6$-$Pro^7$-$Ser^8$-$Pro^9$-$Ser^{10}$-$Arg^{11}$-$Leu^{12}$-$Pro^{13}$-$Gly^{14}$, mit einem Träger-Protein in Gegenwart von Glutaraldehyd konjugiert wird und das resultierende Konjugat einem warmblütigen Tier, jedoch nicht dem Menschen, eingeimpft wird, um einen Antikörper zu erzeugen und diesen zu gewinnen.

11. Immunchemische Analysenreagentien nach Anspruch 10, bei denen das Peptid

H—Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-OH

ist.

12. Immunchemische Analysenreagentien nach Anspruch 7, bei denen die beim Koppeln der Meerrettich-Peroxidase mit dem markiert werdenden Antikörper verwendete Verbindung eine Verbindung der Formel

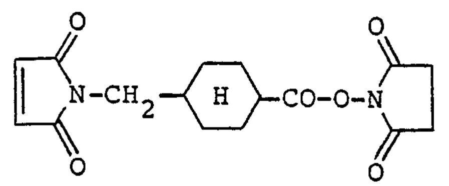

ist.

**Revendications**

1. Dosage immunochimique de la gonadostimuline chorionique humaine à un taux de 0,1 à 2 mUI impliquant l'utilisation d'un anticorps fixé sur un support, d'un antigène et d'un anticorps marqué avec un agent de marquage, caractérisé en ce que ledit anticorps fixé et ledit anticorps marqué sont des anticorps dissemblables dont les déterminants antigéniques ne se chevauchent pas l'un l'autre, en ce que l'anticorps fixé est dirigé contre la sous-variété B de la gonadostimuline chorionique humaine, en ce que ledit agent de marquage est la peroxydase de raifort, et en ce que la peroxydase est couplée à l'anticorps marqué à l'aide d'un composé représenté par la formule:

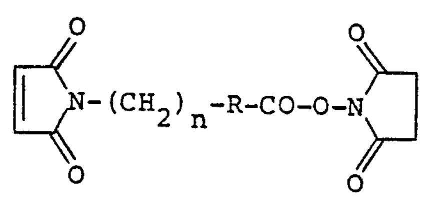

[I]

dans laquelle n est un nombre entier valant de 0 à 5 et R est une liaison chimique ou un résidu divalent d'hydrocarbure saturé ou insaturé, cyclique, à 6 chaînons.

2. Dosage immunochimique selon la revendication 1, dans lequel l'anticorps dirigé contre la sous-variété B de la gonadostimuline chorionique humaine est un anticorps obtenu par la mise en contact d'un fluide corporel contenant un anticorps anti-GCh avec un peptide de formule:

H—$R_1$-Pro-Ser-Asp-Thr-Pro-Ile-Leu-Pro-Gln-OH

dans laquelle $R_1$ est un peptide partiel avec de 1 à 14 résidus d'acides aminés, comprenant le 14-Gly, du peptide $Ala^1$-$Pro^2$-$Pro^3$-$Pro^4$-$Ser^5$-$Leu^6$-$Pro^7$-$Ser^8$-$Pro^9$-$Ser^{10}$-$Arg^{11}$-$Leu^{12}$-$Pro^{13}$-$Gly^{14}$, immobilisé sur un support, et la séparation par élution de l'anticorps anti-GCh spécifiquement absorbé.

3. Dosage immunochimique selon la revendication 2, dans lequel le peptide est

H—Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-OH.

4. Dosage immunochimique selon la revendication 1, dans lequel l'anticorps dirigé contre la sous-variété B de la gonadostimuline chorionique humaine est un anticorps obtenu par la conjugaison d'un peptide de formule:

H—$R_1$-Pro-Ser-Asp-Thr-Pro-Ile-Leu-Pro-Gln-OH

dans laquelle $R_1$ est un peptide partiel de 1 à 14 résidus d'acides aminés comprenant 14-Gly, du peptide $Ala^1$-$Pro^2$-$Pro^3$-$Pro^4$-$Ser^5$-$Leu^6$-$Pro^7$-$Ser^8$-$Pro^9$-$Ser^{10}$-$Arg^{11}$-$Leu^{12}$-$Pro^{13}$-$Gly^{14}$, avec une protéine support, en présence de glutaraldéhyde, et l'inoculation du produit conjugué résultant dans un animal à sang chaud autre que l'homme, pour produire un anticorps, et la récupération de ce dernier.

5. Dosage immunochimique selon la revendication 4, dans lequel le peptide est

H—Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-OH.

6. Dosage immunochimique selon la revendication 1, dans lequel le composé utilisé dans le couplage de la peroxydase de raifort à l'anticorps marqué est un composé représenté par la formule:

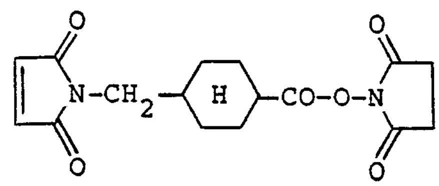

7. Réactifs du dosage immunochimique à utiliser dans un dosage de la gonadostimuline chorionique humaine à un taux de 0,1 à 2 mUI selon la revendication 1, qui comprennent:

a) un réactif constitué d'un produit conjugué qui est préparé par le couplage d'une peroxydase de raifort à un anticorps grâce à l'utilisation du composé de formule:

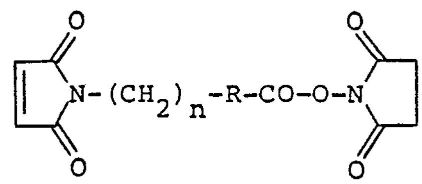

[I]

dans laquelle n est un nombre entier valant de 0 à 5 et R est une liaison chimique ou un résidu divalent d'hydrocarbure saturé ou insaturé, cyclique, à six chaînons, et

(b) un anticorps fixé sur un support, dont le déterminant antigénique ne chevauche pas celui de l'anticorps à coupler à la peroxydase et qui est dirigé contre la sous-variété B de gonadostimuline chorionique humaine.

8. Réactifs du dosage immunochimique selon la revendication 7, dans lesquels l'anticorps dirigé contre la sous-variété B de la gonadostimuline chorionique humaine est un anticorps obtenu par la mise en contact d'un fluide corporel contenant un anticorps anti-GCh avec un peptide de formule:

H—$R_1$-Pro-Ser-Asp-Thr-Pro-Ile-Leu-Pro-Gln-OH

dans laquelle $R_1$ est un peptide partiel de 1 à 14 résidus d'acides aminés, comprenant 14-Gly, du peptide $Ala^1$-$Pro^2$-$Pro^3$-$Pro^4$-$Ser^5$-$Leu^6$-$Pro^7$-$Ser^8$-$Pro^9$-$Ser^{10}$-$Arg^{11}$-$Leu^{12}$-$Pro^{13}$-$Gly^{14}$, immobilisé sur un support, et la séparation par élution de l'anticorps anti-GCh spécifiquement absorbé.

9. Réactifs du dosage immunochimique selon la revendication 8, dans lesquels le peptide est

H—Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-OH.

10. Réactifs du dosage immunochimique selon la revendication 7, dans lesquels l'anticorps dirigé contre la sous-variété B de gonadostimuline chorionique humaine est un anticorps obtenu par la conjugaison d'un peptide de formule:

H—$R_1$-Pro-Ser-Asp-Thr-Pro-Ile-Leu-Pro-Gln-OH

dans laquelle $R_1$ est un peptide partiel de 1 à 13 résidus d'acides aminés, comprenant 14-Gly, du peptide $Ala^1$-$Pro^2$-$Pro^3$-$Pro^4$-$Ser^5$-$Leu^6$-$Pro^7$-$Ser^8$-$Pro^9$-$Ser^{10}$-$Arg^{11}$-$Leu^{12}$-$Pro^{13}$-$Gly^{14}$ avec une protéine support, en présence de glutaraldéhyde, et l'inoculation du produit conjugué résultant dans un animal à sang chaud autre que l'homme pour produire un anticorps, et la récupération de ce dernier.

11. Réactifs du dosage immunochimique selon la revendication 10, dans lesquels le peptide est

H—Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-OH.

12. Réactifs du dosage immunochimique selon la revendication 7, dans lesquels le composé utilisé dans le couplage de la peroxydase de raifort à l'anticorps marqué est un composé représenté par la formule:

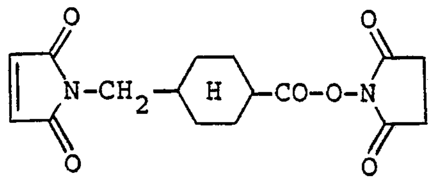

Fig. 1

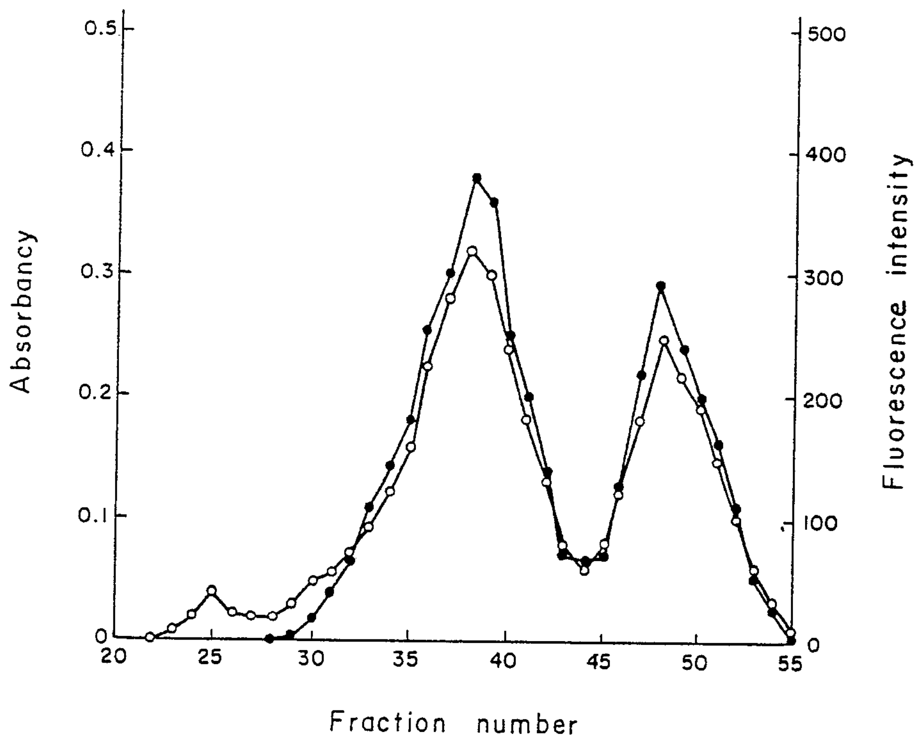

0.5
0.4
0.3
0.2
0.1
0
Absorbancy
500
400
300
200
100
0
Fluorescence intensity
20
25
30
35
40
45
50
55
Fraction number

*Fig. 2*

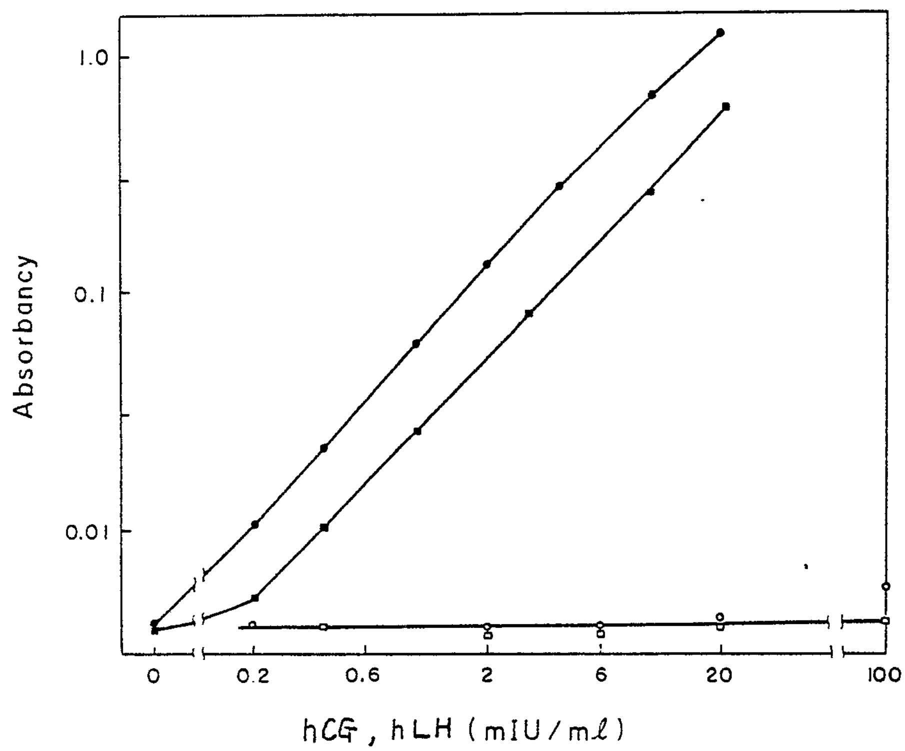

*Fig. 3*